# EUROPEAN PATENT SPECIFICATION

(11) **EP 1 751 272 B1**
(45) Date of publication and mention of the grant of the patent: **17.12.2008**
(21) Application number: 04791706.7
(22) Date of filing: 15.10.2004
(51) Int. Cl.: C12N 1/20, C12P 17/18, A61K 31/535, C12R 1/465

(54) **PRODUCTION OF TACROLIMUS (FK-506) USING NEW STREPTOMYCES SPECIES**
HERSTELLUNG VON TACROLIMUS (FK-506) UNTER VERWENDUNG NEUER STREPTOMYCES-SPEZIES
PRODUCTION DE TACROLIMUS (FK-506) AU MOYEN DE NOUVELLES ESPECES DE STREPTOMYCES

(30) Priority: 17.10.2003 IN DE01284200
(43) Date of publication of application: 14.02.2007
(73) Proprietor: Ranbaxy Laboratories Limited, Gurgaon - 122001, Haryana (IN)
(72) Inventor: KUMAR, Parveen, Ambala, Haryana 134112 (IN); SHARMA, Sunita, New Delhi, Delhi 110091 (IN); SHUKLA, Aniruddha, New Delhi, Delhi 110070 (IN); KUMAR, Sudeep, Ghaziabad, Uttar Pradesh 201011 (IN); MAURYA, Raj Kumar, Ghaziabad, Uttar Pradesh 201010 (IN); KATIAL, Vikas, Noida, Uttar Pradesh 201303 (IN); MITRA, Ashoke, New Delhi, Delhi 110044 (IN); GIGRAS, P., Shri Jagdish Chander Jitender Kumar, Moradabad, Uttar Pradesh 244001 (IN)
(74) Representative: Cronin, Brian Harold John
(86) International application number: PCT/IB2004/003380
(87) International publication number: WO 2005/038009

(56) References cited:
- US-A- 4 894 366
- US-A- 5 116 756
- US-A- 5 194 378
- US-A- 5 624 842
- MOTAMEDI H ET AL: "The biosynthetic gene cluster for the macrolactone ring of the immunosuppressant FK506" EUROPEAN JOURNAL OF BIOCHEMISTRY, vol. 256, no. 3, 1998, pages 528-534, XP000906738 ISSN: 0014-2956
- REYNOLDS K A ET AL: "Rapamycin, FK506, and ascomycin-related compounds" DRUGS AND THE PHARMACEUTICAL SCIENCES, vol. 82, no. 82, 1997, pages 497-520, XP000906777 ISSN: 0360-2583

## Description

### Field of the Invention

The field of the invention relates to a new microorganism *Streptomyces glaucescens* MTCC 5115 and a process for producing the immunosuppressant agent, tacrolimus (FK-506) utilizing the new microorganism *Streptomyces glaucescens* MTCC 5115. The invention also relates to a process for producing pharmaceutical compositions that include the tacrolimus (FK-506) and that can be used for treatment or prevention of the transplantation rejection of organs or tissues such as heart, kidney, liver, medulla ossium, skin, etc.

### Background of the Invention

In recent past, a variety of macrolide compounds have been used as immunosuppressive agents for prevention of graft rejection in bone marrow and organ transplantations and in the treatment of various auto-immune diseases. One widely accepted immunosuppressant for prevention of graft rejection is cyclosporine. Although cyclosporine is widely used in immunosuppressive therapy, its usage particularly in higher concentrations is often accompanied by side effects like nephrotoxicity, hepatotoxicity and central nervous system disorders.

In light of this, newer safer drugs exhibiting less side effects and more potency are constantly being searched. As a result, new compounds like rapamycin and its derivative ascomycin or synthetic analogs collectively called as ascomycins have been discovered as immunosuppressants. Among Ascomycins, FK-506, commonly called as tacrolimus of Formula I, has been demonstrated to be potent immunosuppressant.

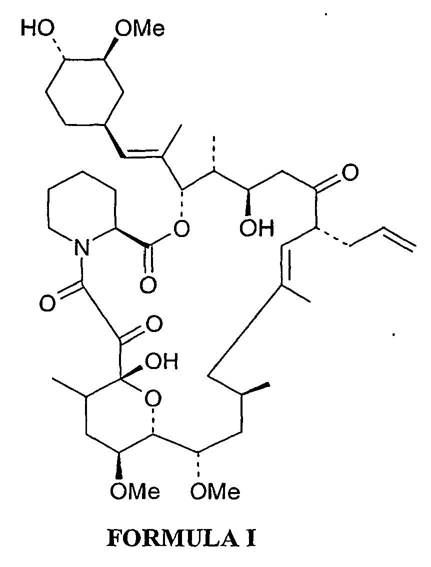

Furthermore tacrolimus and its derivatives have been shown to be effective in treating a number of diseases like Asthma (PCT Patent Application WO 90/14826), inflammatory and hyperproliferative skin disease and cutaneous manifestations of immunologically induced illness (European Patent No 315,978). In spite of its extreme usefulness and higher potency for prevention of graft rejection, tacrolimus production has its own drawbacks such as low productivity due to several related compounds being produced during the process.

Several processes have been reported for the production of tacrolimus for example, in U.S. Patent Nos. 4,894,366; 5,116,756; 5,264,355; 5,496,727 and 5,624,842. In general, these processes involve the use of various strains of Streptomyces species for the production of tacrolimus.

The product obtained by the reported processes, also result in several undesired related compounds. In order to increase the productivity of tacrolimus with simultaneous reduction in several related impurities produced during the process of fermentation, we embarked upon isolating a new strain of the *Streptomyces* species capable of producing tacrolimus. We have surprisingly found that the new strain results only in small amounts of related compounds during bioconversion.

### Summary of the Invention

In one general aspect there is provided a new microorganism, *Streptomyces glaucescens* MTCC 5115 in biologically pure form.

In another general aspect there is provided a new microorganism, *Streptomyces glaucescens* MTCC 5115 capable of producing tacrolimus using fermentation. The produced compound possesses and displays all of the physical and chemical characteristics of tacrolimus (FK-506) as described in EPO Publication No. 0184162.

In another general aspect there is provided a process for producing the immunosuppressant, tacrolimus. The process includes the step of culturing a strain of *Streptomyces glaucescens* MTCC 5115, using conventional methods, under submerged aerobic fermentation conditions in an aqueous carbohydrate medium, containing a nitrogen nutrient, for a sufficient time to produce the product tacrolimus.

In another aspect there is provided a process for producing a pharmaceutical composition that includes a therapeutically effective amount of tacrolimus and one or more pharmaceutically acceptable carriers, excipients or diluents.

The details of one or more embodiments of the inventions are set forth in the description below. Other features, objects and advantages of the inventions will be apparent from the description and claims.

### Detailed Description of the Invention

The inventors have found a new microorganism *Streptomyces glaucescens* MTCC 5115, which has been isolated from soil samples collected from Narnaul, (Haryana), India.

*Streptomyces glaucescens,* isolated from the soil samples collected from Narnaul, Haryana, India, has been deposited under Budapest treaty under accession number MTCC 5115 at Microbial Type Culture Collection (MTCC), Chandigarh, India.

The inventors have also found that the new microorganism *Streptomyces glaucescens* MTCC 5115 under appropriate fermentation conditions is capable of producing tacrolimus to a high extent, while the undesired related compounds are obtained only in small amounts during the bioconversion.

*Streptomyces glaucescens* MTCC 5115 has the following morphological, cultural, biological and physiological characteristics.

### (A) Morphological & cultural characteristics of Streptomyces glaucescens MTCC 5115

The cell wall analysis was performed by the methods of Becker et al. Analysis of whole cell hydrolysates of the strain showed the presence of LL-diaminopimelic acid. Accordingly, the cell wall of this strain is believed to be of type-I (Becker, B. et al., Applied Microbiology, 12 421-423, 1964).

Morphological observations were made in accordance to methods described by Shirling & Gottlieb (Shirling E.B. and D. Gottlieb, International Journal of Systemic Bacteriology, 16, 313-340, 1966). The culture was grown at 28°C for 11 days on yeast extract, malt extract agar (YMA). The spore chain morphology was found to be of spiral/retina type, substrate mycelium was off-white and aerial mycelium was gray orange. Colonies were opaque, raised in the center with irregular edges, rough and hard in consistency.

Microscopic studies and cell wall analysis of the strain reveal that this strain belongs to the genus *Streptomyces.* However, the results show some stark differences between the new isolate and the known tacrolimus (FK-506) producers (Tables 1 and 2).

Cultural characteristics were observed on various kinds of solid media, for example oat meal agar, YMA, tap water agar, basal mineral agar, inorganic salt starch mineral agar, glycerol asparagine agar and Czapek agar. The culture was incubated at 28°C for 11 days. The culture exhibited very good growth on Czapek's agar, nutrient agar, potato dextrose agar and glucose asparagine agar. On basal mineral salt agar, tap water agar and oat meal agar the growth was sparse. Soluble pigment production was observed on YMA, in contrast to the Merck strain ATCC 55098, a known producer of tacrolimus (FK-506). Coloration of vegetative mycelium of *Streptomyces glaucescens* MTCC 5115 ranges from white to off-white whereas in case of *Streptomyces tsukubaensis 9993,* another FK-506 producer strain, it ranges from pale pink to reddish orange to pale brown (Tables 1 and 2).

**Table 1**

| **Comparative Morphological and Cultural Properties of *Streptomyces glaucescens*** | | | | |
|---|---|---|---|---|
| **MTCC 5115 with known FK-506 producers** | | | | |
| S.No. | Test | Strain | | |
| | | *Streptomyces* | *Streptomyces* | *Streptomyces* |
| | | *glaucescens* | *tsukubaensis* | *species* |
| | | MTCC 5115 | ATCC 9993 | ATCC 55098 |
| 1 | Cell wall aminoacids | LL-DAP | LL-DAP | LL-DAP |
| 2 | Cell wall sugars | No diagnostic sugars | Data not available | Glucose |
| 3 | Cell wall type | Type 1 | Type 1 | Type 1 |
| 4 | Spore chain morphology | Spiral/Retina | Rectiflexibles | Straight chain |
| 5 | Aerial mycelium (YME 20 days) | Gray orange | Light gray | Yellowish white |
| 6 | Diffusible pigment | Pale yellow | Dull reddish orange | Negative |
| 7 | Diffusible pigment Produced | Positive | Positive | Negative |
| 8 | Color series | Off white | Gray | White or yellow |
| 9 | Gram's reaction | Gram positive | Gram positive | Gram positive |
| 10 | Acid fast staining | Negative | Data not available | Data not available |

**Table 2**

| **Biological and Physiological Characteristics of** | | | | |
|---|---|---|---|---|
| ***Streptomyces glaucescens* MTCC 5115** | | | | |
| S.No | Test | Strain | | |
| | | *Streptomyces glaucescens* MTCC 5115 | *Streptomyces tsukubaensis* ATCC 9993 | *Streptomyces species* ATCC 55098 |
| 1 | Melanin pigment | Positive | Positive | Negative |
| 2 | Nitrate reduction | Negative | Negative | Data not available |
| 3 | Growth on 7% NaCl | Negative | <=3% | Data not available |
| 4 | Xanthine | Positive | Data not available | Data not available |
| 5 | Optimum growth temperature | 28g°C | 28°C | 27°C |

### (B) Biological & Physiological Properties of Streptomyces glaucescens MTCC

The results are shown in Table 2. Melanoid pigment formation in Streptomyces glaucescens MTCC 5115 was positive whereas it was found to be negative in the earlier known producer of FK-506. Temperature range for growth on YMA was found to be 22-35oC, with optimum temperature at 28oC.

The sugar utilization pattern of MTCC 5115 vis-à-vis known FK-506 producing strains showed ready utilization of fructose, galactose, glucose, mannitol, sucrose; poor utilization of rhamnose and no utilization of arabinose, m-inositol, raffinose, salicin and xylose (Table 3). Streptomyces glaucescens MTCC 5115 sparingly utilized phenylalanine and proline but readily utilized histidine.

**Table 3**

| **Sugar Utilization Pattern of *Streptomyces glaucescens* MTCC 5115*** | | | | |
|---|---|---|---|---|
| S.No. | Sugars | Strain | | |
| | | *Streptomyces* | *Streptomyces* | *Streptomyces* |
| | | *glaucescens* | *tsukubaensis* | *species* |
| | | MTCC 5115 | ATCC 9993 | ATCC 55098 |
| 1 | Arabinose | Negative | Negative | Poor positive |
| 2 | Fructose | Positive | Negative | Moderate utilization |
| 3 | Galactose | Positive | Negative | Data not available |
| 4 | Glucose | Positive | Doubtful | Moderate |
| 5 | Meso-Inositol | Negative | Negative | Negative |
| 6 | Mannitol | Positive | Negative | Poor utilization |
| 7 | Raffinose | Negative | Negative | Poor utilization |
| 8 | Rhamnose | Positive weak | Negative | Poor utilization |
| 9 | Salicin | Negative | Doubtful | Data not available |
| 10 | Sucrose | Positive | Doubtful | Negative |
| 11 | Xylose | Negative | Negative | Negative |

| | | | | |
|---|---|---|---|---|
| * Pridham, T.G. and Gottlieb D.; The utilization of carbon compounds by some Actinomycetales as an aid for species determination. J. Bacteriology 56, 107-117, 1948. | | | | |

Comparison of various morphological, biological and physiological characteristics showed that *Streptomyces glaucescens* MTCC 5115 is a different strain than the earlier known producers of FK-506. A comparison of restriction fragment analysis of 16s r DNA of *Streptomyces glaucescens* MTCC 5115 with known producers of FK-506 *(Streptomyces tsukubaensis 9993)* showed a clear cut difference thereby further confirming it to be a different strain.

Accordingly, a comparison of this strain was made with various *Streptomyces species* in light of the published description (Bergy's Manual of Determinative Bacteriology 8th edition, 1974). As a result of the comparison, the strain *Streptomyces glaucescens* MTCC 5115 is considered to have close resemblance to *Streptomyces glaucescens.*

The inventors also have developed process for the production of tacrolimus using the new microorganism *Streptomyces glaucescens* MTCC 5115. It also includes use of any of its mutants/ variants which are capable of producing tacrolimus substance including natural as well as artificial mutants which can be obtained from the described organism by conventional means such as irradiation by UV, X-rays, gamma rays and treatment with N-Methyl-N'-nitro-N-nitrosoguanidine (NTG), ethyl methanesulphonate (EMS), methyl methanesulphonate (MMS) and the like or genetic manipulations.

The process involves culturing (fermenting) *Streptomyces glaucescens* MTCC 5115, under submerged aerobic conditions in an aqueous nutrient medium, containing sources of assimilable carbon and nitrogen; and isolating the tacrolimus from said medium.

Examples of sources of carbon in the nutrient medium include carbohydrates such as glucose, xylose, galactose, dextrose, glycerol, starch, dextrin, maltose, polyethylene glycol, soybean oil and the like. Other sources may include rhamnose, raffinose, arabinose, mannose, salicin, sodium succinate, and the like.

Examples of sources of nitrogen include yeast extract, soy peptone, soybean meal, cottonseed meal, corn steep liquor, wheat peptone, maize gluten, milk powder, wheat germ, and the like.

The carbon and nitrogen sources, though advantageously employed in combination, need not be used in their pure form.

In addition, mineral salts of calcium, magnesium, sodium, cobalt, copper and potassium can also be added to the nutrient medium. If necessary, especially when the culture medium foams seriously, a defoaming agent, such as liquid paraffin, fatty oil, plant oil, mineral oil or silicone may be added.

Furthermore, when the growth is carried out in large tanks, vegetative form of the microorganism may be used in order to avoid lag phase in the production of tacrolimus. The vegetative form of the organism may be produced by inoculating the medium with spores or mycelia of the organism from the slant and culturing said inoculated medium, also called the seed medium (stage I) and then transferring the stage I seed into II stage seed medium (stage II). This seed can be used for inoculating the production medium.

During fermentation, the culture grows in the form of pellets and hence it requires low agitation to meet the oxygen demand of the culture. This provides an advantage over the other *Streptomyces species* which grow in the filamentous form. This often causes shortage of oxygen in the culture. In order to increase oxygen supply; agitation rate is increased, which in turn further causes lot of shear of hyphae and activity of the microorganism tend to decrease.

The nutrient medium may be maintained at a pH of about 6-8 at the initiation and termination (harvest). In particular, the pH may be maintained at about 6.8-7.5.

The fermentation may be carried out under submerged aerobic conditions at a temperature of from about 20°C to about 40°C, for example at a temperature from about 24° to about 35°C. The fermentation may be carried out for a period of from about 120 hours to about 400 hours, which may be varied according to fermentation conditions and scales of production. In particular, the production cultures may be incubated for about 240 to 310 hours at 27°C at a pH of about 6.8 -7.5.

Media for culturing and carrying out the fermentation include the following:

| Components | g/L |
|---|---|
| Dextrose | 5.0 - 30.0 |
| Dextrin | 30 - 200 |
| Cotton seed meal | 5.0 - 20.0 |
| Soybean meal | 5.0 - 20.0 |
| Soy peptone | 5.0 - 20.0 |
| Glycerol | 5.0 - 30.0 |
| KH₂PO₄ | 0.2 - 1.5 |
| CaCO₃ | 0.5 - 3.0 |
| Polyethylene glycol | 1.0 - 10.0 |

This culturing media provides an advantage as all plant derived raw materials are used in this and thus it is free from all the Transmissible Spongiform Encephalitis (TSE) /Bovine Spongiform Encephalitis (BSE) issues.

The product tacrolimus obtained can be recovered from the cultural medium by conventional means, e.g. High Pressure Liquid chromatography (HPLC). The tacrolimus substance produced is found in the cultured medium and filtrate, and can be isolated and purified from the mycelium and the filtrate.

Isolating the tacrolimus from the medium includes one or more steps of filtration, centrifugation, concentration, concentration under reduced pressure, lyophilization, acidification, extraction with a suitable solvent, treatment with adsorbents, treatment with resins, purification, and crystallization.

The term 'suitable solvent' includes any solvent or solvent mixture, in which tacrolimus can be solubilized, including, for example, ethyl acetate, methanol, toluene, and the like. It may be treated with absorbents such as activated charcoal, silica gel, alumina, or the like. Examples of resins include anion and cation exchange resins.

The product obtained may be recrystallized one or more times to get higher purity.

The tacrolimus can be administered for the prevention and treatment of the transplantation rejection of organs or tissues such as heart, kidney, liver, medulla ossium, skin, etc. in a warm-blooded animal.

For the purpose of this disclosure, a warm-blooded animal is a member of the animal kingdom possessed of a homeostatic mechanism and includes mammals and birds.

In the following section embodiments are described by way of examples to illustrate the process of invention.

### Example 1: Isolation of Streptomyces glaucescens MTCC 5115

*Streptomyces glaucescens* MTCC 5115 was isolated from soil by using dilution plate technique as described below.

Soil samples were collected from different geographical areas of India in order to isolate new microorganisms producing tacrolimus. The samples were air-dried and a pre-treated by heating at 90°C for 1 hour. One gram of the pre-treated sample was taken in a sterile test tube and volume was made up to 10 ml with sterile distilled water. The mixture was blended for 10 seconds and allowed to stand for 1 hour. Serial dilutions were made in pre-sterilized distilled water and plated on asparagine-glycerol medium containing 50 µg/ml each of chloramphenicol and nystatin/cycloheximide. One ml suspension was used for plating. The plates were incubated at 28°C for 7-10 days.

Isolated colonies were picked up on fresh YMA plates. The plates were incubated at 28°C for 10 days. The growth on the plates was picked up on YMA slants and cultured for 10-11 days at 28°C. These slants were stored for further use.

### Example 2: Screening of the isolates

A spore/mycelial suspension of each isolate were prepared with 2.5 ml of 0.85% sodium chloride solution obtained from 11 day old YMA slant. The suspension was used to inoculate 35 ml of sterile KE seed medium as described in U.S. Patent No.5,194,378.

The pH of the seed medium was adjusted to 6.9 before sterilization. The culture was incubated for 44-48 hours on a rotary shaker at 28°C, then 2 ml of the seed culture was used to inoculate 25 ml of FKA production medium and the fermentation was carried out as per the conditions described in U.S. Patent No. 5,194,378.

Methanol extracts of the fermentation broths were tested for antifungal activity against a mutant strain *of Aspergillus terreus* using FK-506 as standard. Twenty two strains showed a clearing zone thereby showing antifungal activity against *Aspergillus terreus.* Extracts of these strains were confirmed for the presence of FK-506 using HPLC. One of the strains, *Streptomyces glaucescens* MTCC 5115, showed a very small peak at the same retention time as that of the standard FK-506.

### Example 3: Restriction fragment analysis of 16s rDNA

A comparison of restriction fragment pattern of 16s rDNA between *Streptomyces glaucescens* MTCC 5115 and *Streptomyces tsukubaensis 9993* was made using different restriction endonucleases (BamH1, Bgl1, Nco1, Sma1, EcoR1, HindIII, EcoRV, Stu1 & Psn AI). These experiments reveal that *Streptomyces glaucescens* MTCC 5115 exhibits a different prototype.

### Example 4: Process optimization

A spore/mycelial suspension of *Streptomyces glaucescens* MTCC 5115 strain was prepared with 2.5 ml of 0.85% sodium chloride solution obtained from 11 day old YMA slant. The seed was grown through Stage I / II for 44-48 hours at 28°C.The composition of the seed medium is given below.

| Seed medium | |
|---|---|
| Ingredients | g per litre |
| Dextrin | 10.0 |
| Dextrose | 1.0 |
| Cottonseed meal | 2.5 |
| Yeast extract | 5.0 |
| Casein enzyme hydrolysate | 5.0 |
| MgSO₄.7H₂O | 0.05 |
| Milk powder | 2.0 |
| Phosphate buffer (0.67M) pH 7.0 | 2 mL |
| NaCl | 0.5 |
| ZnSO₄.7H₂O | 0.001 |
| MnSO₄.4H₂O | 0.005 |
| CaCl₂ | 0.02 |
| FeSO₄.5H₂O | 0.025 |

Two ml of seed culture was used to inoculate production medium having the following composition.

| Production medium | |
|---|---|
| Ingredients | g per litre |
| Dextrose | 5.0 |
| Dextrin | 120.0 |
| Cottonseed meal | 10.0 |
| Soybean meal | 10.0 |
| Soy peptone | 10.0 |
| Glycerol | 10.0 |
| KH₂PO₄ | 0.8 |
| CaCO3 | 1.5 |
| Polyethylene glycol | 1.0 |

The pH of the nutritive medium was adjusted to 7.0-7.2 before sterilization and the medium was sterilized at 121°C for 25 minutes.

The fermentation was carried out for 290-310 hours at 240 rpm, 24°C and analyzed for tacrolimus by HPLC. The tacrolimus (FK-506) peak was very distinct and clear.

Spiking of this HPLC peak using FK-506 working standard confirmed the presence of tacrolimus (FK-506) product in the broth. The highest titer of FK-506 was 5-10 µg/ml after about 300 hours from above mentioned production medium.

### Example 5: Isolation and Purification of FK-506

Six liters of fermentation broth was acidified to pH 4.0, and it was extracted twice with equal volume of ethyl acetate. The ethyl acetate layer was pooled (6 liters) and concentrated to 100 ml at 40°C under vacuum. This was further purified by known techniques of column chromatography and the isolated product was tested for MASS by LCMS.

The analytical results demonstrated that the compound isolated was similar to tacrolimus (FK-506) as described in European Patent No. 184162 and U.S. Patent No. 4,894,366.

## Claims

1. An isolated microorganism *Streptomyces glaucescens* MTCC 5115.

2. A fermentation process for producing tacrolimus (FK-506) of Formula I, 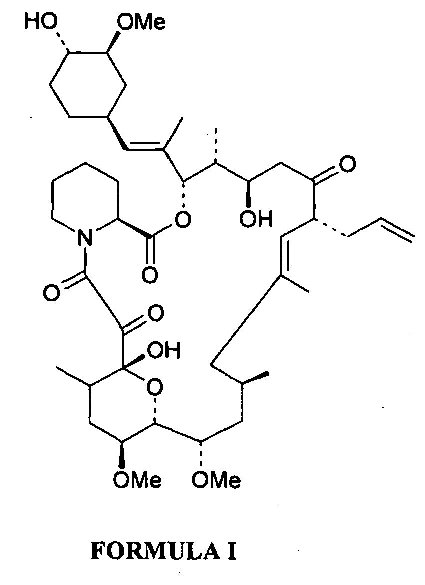 the process comprising using isolated *Streptomyces glaucescens* MTCC 5115.

3. A process for producing tacrolimus, the process comprising the steps of culturing a strain of *Streptomyces glaucescens* MTCC 5115 under submerged aerobic fermentation conditions in an aqueous nutrient medium; and isolating the tacrolimus from said medium.

4. The process of claim 3, wherein the nutrient medium comprises one or more of carbon source, nitrogen source and mineral salts.

5. The process of claim 4, wherein the carbon source is a carbohydrate medium.

6. The process of claim 5, wherein the carbohydrate source comprises one or more of glucose, xylose, galactose, glycerol, dextrose, starch, dextrin, maltose, polyethylene glycol, soybean oil, rhamnose, raffinose, arabinose, mannose, salicin and sodium succinate.

7. The process of claim 4, wherein the nitrogen source comprises one or more of yeast extract, soy peptone, soybean meal, cottonseed meal, corn steep liquor, wheat peptone, maize gluten, milk powder and wheat germ.

8. The process of claim 4, wherein the mineral salts comprises one or more of salts of calcium, magnesium, sodium, cobalt, copper and potassium.

9. The process of claim 4, wherein the nutrient medium further comprises a defoaming agent.

10. The process of claim 3, wherein during fermentation the temperature is maintained from about 20°C to about 40°C.

11. The process of claim 10, wherein the temperature is maintained from about 24° to about 35°C.

12. The process of claim 3, wherein during fermentation pH is maintained from about 6.0-8.0.

13. The process of claim 12, wherein the pH is maintained from about 6.8-7.5.

14. The process of claim 3, wherein the nutrient medium is a liquid culture broth.

15. The process of claim 3, wherein the isolation of tacrolimus comprises one or more steps of filtration, centrifugation, concentration, concentration under reduced pressure, lyophilization, acidification, extraction with a suitable solvent, treatment with adsorbents, treatment with resins, purification, and crystallization.

16. The process of claim 15, further comprising recrystallizing the isolated tacrolimus.

17. The process of claim 16, further comprising forming the product obtained into a finished dosage form.

18. A process for producing a pharmaceutical composition comprising a therapeutically effective amount of tacrolimus (FK-506) and one or more pharmaceutically acceptable carriers, excipients or diluents, comprising producing the tacrolimus by the process of any one of claims 2 to 17.

## Patentansprüche

1. Isolierter Mikroorganismus *Streptomyces glaucescens* MTCC 5115.

2. Fermentationsverfahren zur Herstellung von Tacrolimus (FK-506) der Formel I, 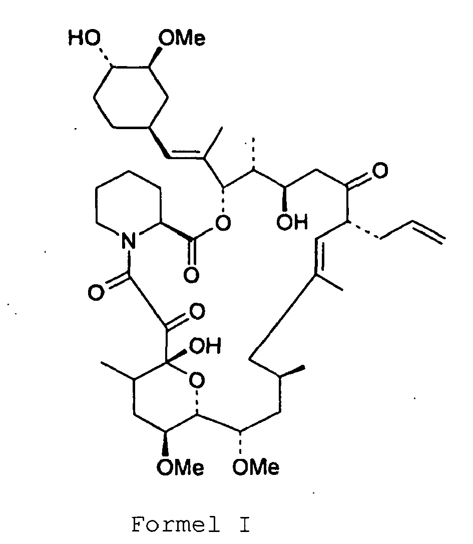 umfassend die Verwendung von isoliertem *Streptomyces glaucescens* MTCC 5115.

3. Verfahren zur Herstellung von Tacrolimus, umfassend die Schritte Kultivieren eines Stamms von *Streptomyces glaucescens* MTCC 5115 in wäßrigem Nährmedium unter Bedingungen aerobischer Submers-Fermentation und Isolieren des Tacrolimus aus diesem Medium.

4. Verfahren nach Anspruch 3, wobei das Nährmedium eine Kohlenstoffquelle, eine Stickstoffquelle und/oder Mineralsalze enthält.

5. Verfahren nach Anspruch 4, wobei die Kohlenstoffquelle ein Kohlenhydratmedium ist.

6. Verfahren nach Anspruch 5, wobei die Kohlenhydratquelle Glukose, Xylose, Galaktose, Dextrose, Stärke, Dextrin, Maltose, Polyethylenglykol, Sojabohnenöl, Rhamnose, Arabinose, Mannose, Salizin und/oder Natriumsuccinat umfasst.

7. Verfahren nach Anspruch 4, wobei die Stickstoffquelle Hefeextrakt, Sojapepton, Sojabohnenmehl, Baumwollsaatmehl, konzentriertes Maisweichwasser (corn steep liquor), Weizenpepton, Maiskleber, Milchpulver und/oder Weizenkeime umfasst.

8. Verfahren nach Anspruch 4, wobei die Mineralsalze Salze des Kalziums, Magnesiums, Natriums, Kobalts, Kupfers und/oder Kaliums umfassen.

9. Verfahren nach Anspruch 4, wobei das Nährmedium ferner einen Entschäumer umfasst.

10. Verfahren nach Anspruch 3, wobei die Temperatur während der Fermentation zwischen etwa 20 °C und etwa 40 °C gehalten wird.

11. Verfahren nach Anspruch 10, wobei die Temperatur zwischen etwa 24 °C und etwa 35 °C gehalten wird.

12. Verfahren nach Anspruch 3, wobei der pH während der Fermentation zwischen etwa 6,0 und 8,0 gehalten wird.

13. Verfahren nach Anspruch 12, wobei der pH zwischen etwa 6,8 und 7,5 gehalten wird.

14. Verfahren nach Anspruch 3, wobei das Nährmedium eine flüssige Kulturbrühe ist.

15. Verfahren nach Anspruch 3, wobei die Isolierung des Tacrolimus die Schritte Filtrieren, Zentrifugieren, Einengen, Einengen unter vermindertem Druck, Gefriertrocknen, Ansäuern, Extrahieren mit einem geeigneten Lösungsmittel, Behandeln mit Adsorptionsmitteln, Behandeln mit Harzen, Reinigen und/oder Kristallisieren umfasst.

16. Verfahren nach Anspruch 15, ferner das Umkristallisieren des isolierten Tacrolimus umfassend.

17. Verfahren nach Anspruch 16, ferner die Umformung des erhaltenen Produkts in eine fertige Darreichungsform umfassend.

18. Verfahren zur Herstellung einer pharmazeutischen Zusammensetzung, enthaltend eine therapeutisch wirksame Menge von Tacrolimus (FK-506) und ein oder mehrere pharmazeutisch zulässige Träger, Hilfsstoffe oder Verdünnungsmittel, umfassend die Herstellung des Tacrolimus nach einem Verfahren nach einem beliebigen der Ansprüche 2 bis 17.

## Revendications

1. Micro-organisme *Streptomyces glaucescens* MTCC 5115 isolé.

2. Procédé de fermentation pour produire du tacrolimus (FK-506) de formule I 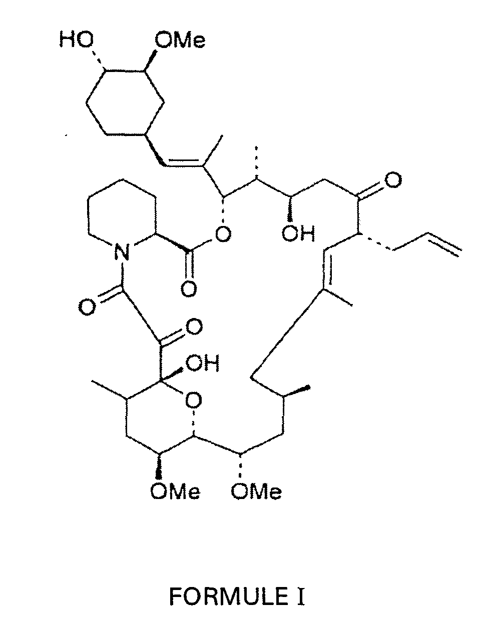 le procédé consistant à utiliser *Streptomyces glaucescens* MTCC 5115 isolé.

3. Procédé pour produire du tacrolimus, le procédé comprenant les étapes de mettre en culture une souche de *Streptomyces glaucescens* MTCC 5115 sous des conditions de fermentation aérobiques submergées dans un milieu nutritif aqueux ; et à isoler le tacrolimus dudit milieu.

4. Procédé de la revendication 3, dans lequel le milieu nutritif comprend un ou plusieurs d'une source de carbone, d'une source d'azote et de sels minéraux.

5. Procédé de la revendication 4, dans lequel la source de carbone est un milieu glucidique.

6. Procédé de la revendication 5, dans lequel la source glucidique comprend un ou plusieurs du glucose, xylose, galactose, glycérol, dextrose, amidon, dextrine, maltose, polyéthylèneglycol, huile de soja, rhamnose, raffinose, arabinose, mannose, salicine et succinate de sodium.

7. Procédé de la revendication 4, dans lequel la source d'azote comprend un ou plusieurs d'extrait de levure, peptone de soja, farine de soja, tourteau de graines de coton, extrait soluble de maïs, peptone de blé, gluten de maïs, lait en poudre et germe de blé.

8. Procédé de la revendication 4, dans lequel les sels minéraux comprennent un ou plusieurs des sels de calcium, magnésium, sodium, cobalt, cuivre et potassium.

9. Procédé de la revendication 4, dans lequel le milieu nutritif comprend de plus un agent antimousse.

10. Procédé de la revendication 3, dans lequel, durant la fermentation, la température est maintenue entre environ 20°C à environ 40°C.

11. Procédé de la revendication 10, dans lequel la température est maintenue entre environ 24°C à environ 35°C.

12. Procédé de la revendication 3, dans lequel, durant la fermentation, le pH est maintenu entre environ 6,0-8,0.

13. Procédé de la revendication 12, dans lequel le pH est maintenu entre environ 6,8-7,5.

14. Procédé de la revendication 3, dans lequel le milieu nutritif est un bouillon de culture liquide.

15. Procédé de la revendication 3, dans lequel l'isolement de tacrolimus comprend une ou plusieurs étapes de filtration, centrifugation, concentration, concentration sous pression réduite, lyophilisation, acidification, extraction avec un solvant approprié, traitement avec des adsorbants, traitement avec des résines, purification et cristallisation.

16. Procédé de la revendication 15, consistant de plus à recristalliser le tacrolimus isolé.

17. Procédé de la revendication 16, consistant de plus à former le produit obtenu sous une forme posologique finie.

18. Procédé pour produire une composition pharmaceutique comprenant une quantité thérapeutiquement efficace de tacrolimus (FK-506) et un ou plusieurs supports, excipients ou diluants pharmaceutiquement acceptables, consistant à produire le tacrolimus par le procédé d'une quelconque des revendications 2 à 17.
